(19) Europäisches Patentamt
European Patent Office
Office européen des brevets

(11) EP 1 570 861 A2

(12) # EUROPÄISCHE PATENTANMELDUNG

(43) Veröffentlichungstag:
**07.09.2005 Patentblatt 2005/36**

(51) Int Cl.7: **A61K 45/06**, A61P 11/00

(21) Anmeldenummer: **05008960.6**

(22) Anmeldetag: **03.05.2000**

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **12.05.1999 DE 19921693**

(62) Dokumentnummer(n) der früheren Anmeldung(en) nach Art. 76 EPÜ:
**03008310.9 / 1 327 452**
**00929478.6 / 1 178 832**

(71) Anmelder: **Boehringer Ingelheim Pharma GmbH & Co.KG**
**55216 Ingelheim am Rhein (DE)**

(72) Erfinder:
- **Bozung, Karlheinz**
  **verstorben (DE)**
- **Jung, Helga**
  **67061 Ludwigshafen (DE)**
- **Pairet, Michel**
  **88400 Biberach (DE)**
- **Reichl, Richard**
  **55435 Gau-Algesheim (DE)**
- **Walland, Alexander**
  **55218 Ingelheim am Rhein (DE)**

Bemerkungen:
Diese Anmeldung ist am 23 - 04 - 2005 als Teilanmeldung zu der unter INID-Kode 62 erwähnten Anmeldung eingereicht worden.

(54) **Neuartige Arzneimittelkompositionen auf der Basis von anticholinergisch wirksamen Verbindungen und B-Mimetika**

(57) Die vorliegende Erfindung betrifft neurtige Arzneimittelkompositionen auf der Basis von anticholinergisch langwirksamen Verbindungen und langwirksamen β-Mimetika, Verfahren zu deren Herstellung und deren Verwendung bei der Therapie von Atemwegserkrankungen.

EP 1 570 861 A2

## Beschreibung

Die vorliegende Erfindung betrifft neurtige Arzneimittelkompositionen auf der Basis von anticholinergisch langwirksamen Verbindungen und langwirksamen β-Mimetika, Verfahren zu deren Herstellung und deren Verwendung bei der Therapie von Atemwegserkrankungen.

Hintergrund der Erfindung

Aus dem Stand der Technik ist bekannt, daß β-Mimetika sowie Anticholinergika als Bronchospamolytika zur Behandlung obstruktiver Atemwegserkrankungen - wie z.B. des Asthmas - erfolgreich eingesetzt werden können. Stoffe mit β-sympathomimetischer Wirksamkeit - wie z.B. der ebenfalls aus dem Stand der Technik bekannte Wirkstoff Formoterol - können bei der Verabreichung am Menschen jedoch mit unerwünschten Nebenwirkungen verbunden sein.

Als zentrale Wirkungen können allgemeine Unruhe, Erregung, Schlaflosigkeit, Angst, Fingerzittern, Schweißausbrüche und Kopfschmerzen auftreten. Dabei schließt die inhalative Anwendung diese Nebenwirkungen nicht aus, sie sind im allgemeinen jedoch etwas geringer als nach peroraler oder parenteraler Anwendung.

Die Nebenwirkungen der β-Sympathomimetika bei der Anwendung als Asthmamittel beruhen aber vor allem auf dem mehr oder weniger ausgeprägten β1-stimulierden Wirkungen am Herzen. Sie erzeugen Tachycardie, Herzklopfen, Angina-pectorisartige Beschwerden sowie Arrhytmien [P.T. Ammon (Hrsg.), Arzneimittelnebenwirkungen und -wechselwirkungen, Wissenschaftliche Verlagsgesellschaft, Stuttgart 1986,S. 584].

Beschreibung der Erfindung

Überraschenderweise wurde nun gefunden, daß durch die Kombination eines langwirksamen β-Sympathomimetikmus mit einem langwirksamen Anticholinergikum die oben erwähnten Nebenwirkungen deutlich reduziert werden können.

Völlig überraschend konnte dabei ebenfalls gefunden werden, daß sich die bronchospasmolytische Wirkung des langwirksamen Anticholinergikums und des langwirksamen β-Mimetikums in überadditiver Wirkung verstärken.

Mit der erfindungsgemäßen Wirkstoffkombination kann somit eine deutlich verbesserte Wirksamkeit - gegenüber den aus dem Stand der Technik bekannten Einzelsubstanzen und Kombinationen - sowohl bei COPD als auch bei Asthma erwartet werden.

Als langwirksame β-Mimetika können in der erfindungsgemäßen Wirkstoffkombination vorzugsweise folgende Wirkstoffe eingesetzt werden: Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tofubuterol, 4-Hydroxy-7-[2-{[2-{[3-(2-phenylethoxy)propyl]sulfonyl}ethyl]-amino}ethyl]-2(3H)-benzothiazolon,
1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol,
1-[2H-5-Hydroxy-3-oxo-4H-1,4-benzoxazin-8-yl]-2-[3-(4-N,N-dimethylaminophenyl)-2-methyl-2-propylamino]ethanol,
1-[2H-5-Hydroxy-3-oxo-4H-1,4-benzoxazin-8-yl]-2-[3-(4-methoxyphenyl)-2-methyl-2-propylamino]ethanol,
1-[2H-5-Hydroxy-3-oxo-4H-1,4-benzoxazin-8-yl]-2-[3-(4-n-butyloxyphenyl)-2-methyl-2-propylamino]ethanol,
1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol,
5-Hydroxy-8-(1-hydroxy-2-isopropylaminobutyl)-2H-1,4-benzoxazin-3-(4H)-on, 1-(4-Amino-3-chloro-5-trifluormethylphenyl)-2-*tert*.-butylamino)ethanol oder 1-(4-Ethoxycarbonylamino-3-cyano-5-fluorophenyl)-2-(*tert*.-butylamino)ethanol,
gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Bevorzugt werden als langwirksame β-Mimetika in der erfindungsgemäßen Wirkstoffkombination eingesetzt Formoterol, Salmeterol,
4-Hydroxy-7-[2-{[2-{[3-(2-phenylethoxy)propyl]sulfonyl}ethyl]-amino}ethyl]-2(3H)-benzothiazolon,
1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol,
1-[2H-5-Hydroxy-3-oxo-4H-1,4-benzoxazin-8-yl]-2-[3-(4-N,N-dimethylaminophenyl)-2-methyl-2-propylamino]ethanol,
1-[2H-5-Hydroxy-3-oxo-4H-1,4-benzoxazin-8-yl]-2-[3-(4-methoxyphenyl)-2-methyl-2-propylamino]ethanol,
1-[2H-5-Hydroxy-3-oxo-4H-1,4-benzoxazin-8-yl]-2-[3-(4-n-butyloxyphenyl)-2-methyl-2-propylamino]ethanol oder
1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol,
gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt gelangen in den erfindungsgemäßen Arzneimittelkompositionen als β-Mimetikum Formoterol oder Salmeterol, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze zur Anwendung.

Wie vorstehend genannt, können die langwirksamen β-Mimetika in Form ihrer physiologisch und pharmakologisch verträglichen Salze überführt und eingesetzt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden.

Unter dem Blickwinkel der überadditiven bronchospasmolytischen Wirkung wird inbesondere das Fumarat des Formoterols (abgekürzt mit Formoterol FU) als langwirksames β-Mimetikum bevorzugt. - Dabei kann der Wirkstoff Formoterol als Enantiomeren- bzw. Diastereomerenmischung oder in Form der einzelnen Enantiomere/Diastereomere eingesetzt werden. Von erfindungsgemäß gleichrangig bevorzugter Bedeutung kann als langwirksames β-Mimetikum Salmeterol zum Einsatz kommen, gegebenenfalls in Form seiner Racemate, Enantiomere, von denen das (R)-Enantiomere höchst bevorzugt ist, sowie gegebenenfalls seiner pharmakologisch unbedenklichen Säureadditionssalze.

Als langwirksame Anticholinergika eignen sich grundsätzlich bereits aus dem Stand der Technik bekannte Verbindungen, wie Glycopyrroniumbromid sowie Ester bi- und tricyclischer Aminoalkohole, wie sie aus der Europäischen Offenlegungsschrift 0 418 716 und der internationalen Patentanmeldung WO 92/16528 bekannt sind und auf die hiermit vollinhaltlich Bezug genommen wird.

Bevorzugt kommen im Rahmen der Erfindung als langwirksame Anticholinergika Glycopyrroniumbromid sowie als Ester bi- und tricyclischer Aminoalkohole die Verbindungen der Formel (I) in Betracht,

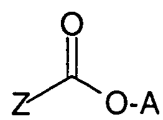

(I)

worin

A für einen Rest der allgemeinen Formel (II)

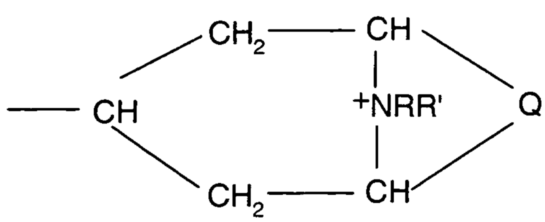

(II)

steht,
in dem

Q eine der zweibindigen Gruppen $-CH_2-CH_2-$, $-CH_2-CH_2-CH_2-$, $-CH=CH-$, oder

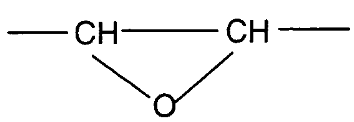

;

R ein gegebenenfalls halogen- oder hydroxysustituierter $C_1$-$C_4$-Alkylrest,
R' ein $C_1$-$C_4$-Alkylrest bedeutet und R und R' gemeinsam auch einen $C_4$-$C_6$-Alkylenrest bilden können, und
der positiven Ladung des N-Atoms ein Äquivalent eines Anions X gegenübersteht,

Z für eine der Gruppen

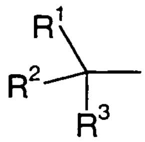 (III) oder 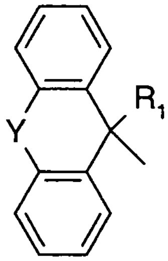 (IV)

steht
worin

Y eine Einfachbindung, ein O- oder S-Atom oder eine der Gruppen $-CH_2-$, $-CH_2-CH_2-$, -CH=CH-, $-OCH_2-$ oder $-SCH_2-$ repräsentiert;

$R^1$ Wasserstoff, OH, $C_1$-$C_4$-Alkoxy oder $C_1$-$C_4$-Alkyl, welches gegebenenfalls durch Hydroxy substituiert sein kann;

$R^2$ ein Thienyl-, Phenyl-, Furyl-, Cyclopentyl- oder Cyclohexylrest, wobei diese Reste auch methylsubstituiert, Thienyl und Phenyl auch fluor- oder chlorsubstitueniert sein können,

$R^3$ Wasserstoff oder ein Thienyl- oder Phenylrest, der gegebenenfalls durch Halogen oder $C_1$-$C_4$-Alkyl substituiert sein kann, bedeuten,

gegebenenfalls in Form ihrer Racemate, ihrer Enantiomere, ihrer Diastereomere und ihrer Gemische.

Besonders bevorzugt gelangen im Rahmen der Erfindung als lankgwirksame Anticholinergika Glycopyrroniumbromid sowie als Ester bi- und tricyclischer Aminoalkohole die Verbindungen der Formel (I) in Betracht, worin

A für einen Rest der allgemeinen Formel (II)

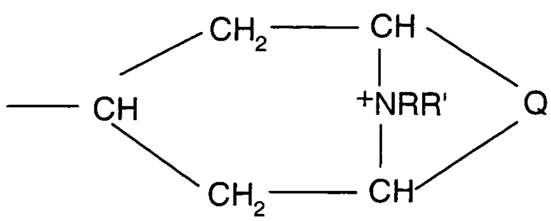 (II)

steht,
in dem

Q eine der zweibindigen Gruppen -CH = CH-, $-CH_2-CH_2-$ oder

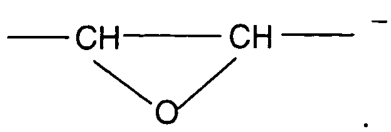

;

R eine gegebenenfalls durch Fluor oder Hydroxy substituierte Methyl-, Ethyl, oder Propyl-Gruppe,
R' Methyl, Ethyl oder Propyl, bevorzugt Methyl, und
der positiven Ladung des N-Atoms ein Äquivalent eines Anions X ausgewählt aus der Gruppe bestehend aus Chlorid, Bromid und Methansulfonat, bevorzugt Bromid gegenübersteht,

Z für eine der Gruppen

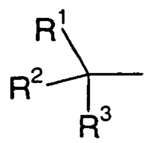 (III) oder 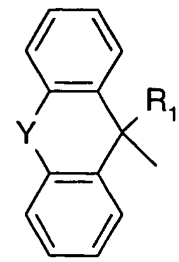 (IV)

steht,
worin

Y eine Einfachbindung, oder ein O-Atom repräsentiert;

$R^1$ Wasserstoff, OH, Methoxy, Ethoxy, Propoxy, Methyl, Ethyl, Propyl, Hydroxymethyl, Hydroxyethyl oder Hydroxyypropyl;

$R^2$ ein Thienyl-, Phenyl-, oder Cyclohexylrest, wobei diese Reste auch methylsubstituiert, Thienyl und Phenyl auch fluor- oder chlorsubstituiert sein können,

$R^3$ Wasserstoff, oder ein Thienyl- oder Phenylrest, der gegebenenfalls durch Fluor, Chlor oder Methyl substituiert sein kann, bedeuten,

gegebenenfalls in Form ihrer Racemate, ihrer Enantiomere, ihrer Diastereomere und ihrer Gemische.

Erfindungsgemäß von besonderer Bedeutung sind Arzneimittelkompositionen, in denen als lankgwirksame Anticholinergika Verbindungen der Formel (I) Verwendung finden, worin

A für einen Rest der allgemeinen Formel (II)

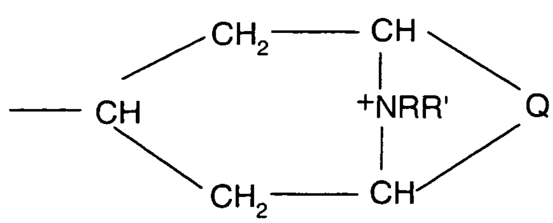

(II)

steht,
in dem

Q eine der zweibindigen Gruppen -CH = CH-, $-CH_2-CH_2-$ oder

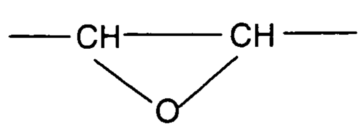

;

R Methyl oder Ethyl;
R' Methyl,
und

der positiven Ladung des N-Atoms ein Äquivalent des Anions X = Bromid gegenübersteht,

Z für eine der Gruppen

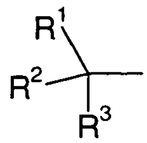 (III) oder 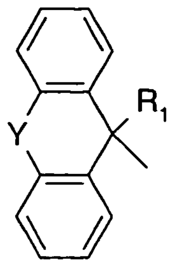 (IV)

steht,

worin

Y ein O-Atom;
$R^1$ Wasserstoff, OH oder Hydroxymethyl;
$R^2$ ein Thienyl-, Phenyl-, oder Cyclohexylrest;
$R^3$ Wasserstoff, Thienyl oder Phenylrest,

gegebenenfalls in Form ihrer Racemate, ihrer Enantiomere, ihrer Diastereomere und ihrer Gemische.

Von den vorstehend genannten Verbindungen sind im Rahmen der vorliegenden Erfindung die der 3-α-Konfiguration besonders bevorzugt.

Die beschriebenen anticholinergen Wirkstoffe können gegebenenfalls in Form der reinen Enantiomeren, deren Mischung bzw. Racemate eingesetzt werden. Besonders bevorzugt wird Tiotropium-Salz - insbesondere das Tiotropiumbromid [(1α,2β,4β,5α,7β)-7-[(Hydroxy-2-thienylacetyl)oxy]-9,9-dimethyl-3-oxa-9-azoniatricyclo[3.3.1 .$0^{2,4}$]nonan-bromid Monohydrat - abgekürzt Tiotropium BR] - als Anticholinergikum eingesetzt.

Als Alkylgruppen (auch soweit sie Bestandteil anderer Reste sind) werden, soweit nicht anders definiert, verzweigte und unverzweigte Alkylgruppen mit 1 bis 4 Kohlenstoffatomen betrachtet. Beispielsweise werden genannt: Methyl, Ethyl, Propyl oder Butyl. Sofern nicht anders genannt, sind von den vorstehend genannten Bezeichnungen Propyl und Butyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl. Gegebenfalls werden zur Bezeichnung der vorstehend genannten Alkylreste auch gängige Abkürzungen wie Me für Methyl, Et für Ethyl etc. verwendet.

Als Alkoxygruppen (auch soweit sie Bestandteil anderer Reste sind) werden, soweit nicht anders definiert, verzweigte und unverzweigte, über ein Sauerstoffatom verbrückte Alkylgruppen mit 1 bis 4 Kohlenstoffatomen betrachtet. Beispielsweise werden genannt: Methoxy, Ethoxy, Propoxy (=Propyloxy) oder Butoxy (=Butyloxy). Auch hier sind, sofern nicht anders genannt, von den vorstehend genannten Bezeichnungen Propoxy und Butoxy sämtliche der möglichen isomeren Formen umfaßt.

Als Alkylengruppen werden verzweigte und unverzweigte Alkylenbrücken mit 4 bis 6 Kohlenstoffatomen betrachtet. Beispielsweise werden genannt: Butylen, Pentylen, Hexylen. Sofern nicht anders genannt, sind von den vorstehend genannten Bezeichnungen Butylen, Pentylen, Hexylen sämtliche der möglichen isomeren Formen umfaßt. Beispielsweise umfaßt die Bezeichnung Butylen die Isomeren n-Butylen, 1-Methylpropylen, 2-Methylpropylen, 1.1-Dimethylethylen, 1.2-Dimethylethylen etc.

Als Halogen wird im allgemeinen Fluor, Chlor, Brom oder Jod bezeichnet.

Als Anion X wird, soweit nicht anders genannt im allgemeinen Fluor, Chlor, Brom, Jod, Methansulfonat, Fumarat, Citrat bezeichnet.

Die erfindungsgemäßen Wirkstoffkompositionen werden vorzugsweise in Form eines Dosierungsaerosols verabreicht - es ist aber auch jede andere Form der parenteralen oder oralen Applikation möglich. Dabei verkörpert die Anwendung von Dosieraerosolen die bevorzugte Anwendungsform insbesondere bei der Therapie obstruktiver Lungenerkrankungen oder bei der Behandlung des Asthmas.

Neben der Anwendung in Dosieraerosolen, die auf der Basis von Treibgasen arbeiten, können die erfindungsgemäßen Wirkstoffkombinationen mittels sog. Verneblern appliziert werden, mit denen Lösungen pharmakologisch aktiver Stoffe unter hohem Druck so versprüht werden, daß Nebel inhalierbarer Teilchen entstehen. Der Vorteil dieser

Vernebler ist, daß auf den Einsatz von Treibgasen völlig verzichtet werden kann.

Überlicherweise sind die zur Inhalation bestimmten Arzneistoffe in einer wässerigen oder ethanolischen Lösung gelöst, wobei je nach den Lösungseigenschaften der Wirkstoffe auch Lösungsmittelgemische aus Wasser und Ethanol geeignet sind.

Derartige Vernebler sind beispielsweise in der PCT-Patentanmeldung WO91/14468 und in der internationalen Patentanmeldung mit dem Aktenzeichen PCT/EP96/04351 beschrieben, auf die hiermit inhaltlich Bezug genommen wird. Bei den dort beschriebenen Verneblern, die auch unter der Bezeichnung Respimat® bekannt sind, werden wirkstoffhaltige Lösungen definierter Volumina unter Anwendung hoher Drucke durch kleine Düsen versprüht, so daß inhalierbare Aerosole mit einer bevorzugten Teilchengröße zwischen 1 und 10, bevorzugt zwischen 2 und 5 Mikrometer entstehen.

Als Lösungsmittel für die Arzneimittelzubereitung sind u.a. Gemische geeignet, die beispielsweise Ethanol als Lösungsmittel enthalten.

Weitere Bestandteile des Lösungsmittels sind neben Wasser gegebenenfalls weitere Cosolventien, ebenfalls kann die Arzneimittelzubereitung Geschmackstoffe und weitere pharmakologische Hilfsstoffe enthalten. Beispiele für Cosolventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Cosolventien sind dazu geeignet, die Löslichkeit von Hilfsstoffen und gegebenenfalls der Wirkstoffe zu erhöhen.

Weitere pharmakologische Hilfsstoffe wie beispielsweise Konservierungsmittel insbesondere Benzalkoniumchlorid, können zugesetzt sein. Die bevorzugte Menge an Konservierungsstoff, insbesondere an Benzalkoniumchlorid liegt zwischen 8 und 12 mg/100 ml Lösung.

Zur Vermeidung von Sprühanomalien können der Wirkstoffkombination Komplexbildner zugesetzt werden. - Geeignete Komplexbildner sind solche die pharmakologisch verträglich sind, insbesondere solche die bereits arzneimittelrechtlich zugelassen sind. Besonders geeignet sind EDTA, Nitrilotriessigsäure, Zitronensäure und Ascorbinsäure wie auch deren Salze. Besonders bevorzugt ist das Dinatriumsalz der Ethylendiamtetraessigsäure.

Der Anteil der gelösten Wirkstoffkombination an der fertigen Arzneimittelzubereitung beträgt zwischen 0.001 und 5 % - vorzugsweise zwischen 0.005 und 3 %, insbesondere 0.01 bis 2 %. Die maximale Konzentration des Arzneistoffes ist abhängig von der Löslichkeit im Lösungsmittel und von der erforderlichen Dosierung zur Erzielung der gewünschten therapeutischen Wirkung.

Folgende Zubereitungsformen seien als Formulierungsbeispiel angeführt:

| **Bestandteile** | **Zusammensetzung in mg/100 ml** |
|---|---|
| Tiotropium bromid | 333.3 mg |
| Formoterol Fumarat | 333.3 mg |
| Benzalkoniumchlorid | 10.0 mg |
| EDTA | 50.0 mg |
| HCl(1n) | ad pH 3.4 |

| **Bestandteile** | **Zusammensetzung in mg/100 ml** |
|---|---|
| Tiotropium bromid | 333.3 mg |
| Salmeterol Xinafoat | 666.6 mg |
| Benzalkoniumchlorid | 10.0 mg |
| EDTA | 50.0 mg |
| HCl(1n) | ad pH 3.4 |

Daneben können die erfindungsgemäßen Wirkstoffkombinationen auch in Form eines Pulvers inhaliert werden. Die Herstellung derartiger Darreichungsformen ist aus dem Stand der Technik bekannt. Sie enthalten neben der Wirkstoffkombination entsprechend der vorliegenden Erfindung pharmakologisch unbedenkliche Träger- oder Hilfsstoffe - wie z.B. mikrokristalline Lactose. Die zur Inhalation vorgesehene Dosis kann beispielsweise in Kapseln abgefüllt werden und hat z.B. folgende Zusammensetzung:

| Bestandteile | Menge |
|---|---|
| Tiotropiumbromid Hydrat | 6 µg |
| Formoterolfumarat x 2 $H_2O$ | 6 µg |
| Lactose Monohydrat | ad 25 mg |

Experimentelle Befunde

Bronchospasmolytische und kardiovaskuläre Wirkung von Tiotropiumbromid, Formoterolfumarat sowie deren Kombination nach inhalativer Applikation wäßriger Lösung mittels Respimat® an narkotisierten Hunden.

Material und Methode

18 mischrassige Hunde mit einem Körpergewicht von 27 bis 32 kg. Haltung in Einzel- bzw. Sammelboxen, pelletiertes Standardfutter, letzte Fütterung ca. 15 Stunden vor Versuchsbeginn, Tränkewasser ad libitum.

Nach Prämedikation mit 2 mg/kg Morphinhydrochlorid i.m. werden 30 mg/kg Pentobarbital-Natrium (Nembutal®) langsam intravenös injiziert. Die Tiere sind mit 1,0 mg/kg i.v. Suxamethonium relaxiert.

Die Tiere werden nach den Intubationen mittels eines Servo-Ventilators 900 C (Fa. Siemens) mit Raumluft und Sauerstoff (4:1) beatmet, Frequenz 15/min., Atemvolumen 6-8 l/min. Für die Registrierung der Atemmechanik wird der Atemfluß mittels Staudruckrohr (Fleisch Nr. 1), das unmittelbar vor dem Orotrachealtubus installiert ist, einem Differentialdruckaufnehmer und -verstärker DCB-4C bestimmt. Ein Katheter wird in der Trachea und ein zweiter (Ballon-)Katheter im Lungenabschnitt des Oesophagus plaziert. Beide werden verbunden mit einem Differenzialdruckaufnehmer und -verstärker zur Bestimmung des transpulmonalen Druckes. Ein Atemmechanik-Rechner (IFD-Mühlheim) ermittelt aus den registrierten Druckwerten den pulmonalen Widerstand (R). Ein Computerprogramm VAS-1 LA (IFD-Mühlheim) bestimmt daraus:

$$\text{Pulmonaler Widerstand} = \frac{\text{max.transpulmonaler Druck}}{\text{Atemfluß}}$$

Die Registrierung der Herzfrequenz erfolgt über EKG (Extremitätenableitung II) und Kardiotachometer.

Nach einer Aquilibrierungsperiode von 30 min. werden kurzfristige Bronchospasmen durch i.v. Injektion von 10 µg/kg Acetylcholinchlorid erzeugt, die 2 - 3 x innerhalb eines ca. 10 min. Abstands wiederholt werden. Die Testsubstanzen Tiotropiumbromid, Formoterolfumarat sowie die Kombination beider Substanzen werden als wässrige Lösungen mit dem BINEB-Zerstäuber (Respimat®) verabreicht. Die Applikation der Kombination erfolgt mit den Einzelkomponenten in Abstand von ca. 1 min. Bei dem BINEB-System erfolgt der Auslösemechanismus am Ende der Exspirationsphase und die zerstäubte Lösung wird in der folgenden Inspirationsphase per Atempumpe in den Tracheobronchialbaum gedrückt.

| Dosierungen | |
|---|---|
| Tiotropium Bromid | 3 und 10 µg/15 µl |
| Formoterol Fumarat | 3 und 10 µg/15 µl |
| Tiotropium Bromid + Formoterol Fumarat | 3 + 3 µg bzw. 10 + 10 µg/15 µl |

Die Tabellen 1 - 6 zeigen die Ausgangswerte und die Werte nach Substanzbehandlung über die Zeit von 180 min. In den Abbildungen 1 - 2 sind die prozentualen Hemmungen der durch ACh-induzierten pulmonalen Widerstandserhöhungen über die Zeit von 180 min. dargestellt.

Ergebnisse

Die Ergebnisse sind in den Tabellen sowie in den Abbildungen dargestellt. 3 und 10 µg Tiotropium Bromid bzw. Formoterolfumarat hemmen den durch intravenöse Injektion von ACh erhöhten Bronchialwiderstand dosisabgestuft und deutlich. Die maximale bronchospasmolytische Wirkung von Formoterol FU tritt mit beiden Dosierungen rasch ein, die von Tiotropium BR verzögert etwa nach 60 min. Die Wirkdauer von Formoterol FU ist vor allem mit den niedrigen Dosierungen verhältnismäßig kurz, die des Tiotropium BR erwartungsgemäß, bis zum Versuchsende (180 min.), anhaltend.

Mit der Kombination von 3 µg Tiotropium Bromid + 3 µg Formoterol FU wird eine ausgeprägte, sehr schnell einsetzende Bronchospasmolyse von 90 % erzielt, die bis zum Versuchsende nahezu unverändert anhält. Die protektive Wirkung der Kombination übertrifft die der Einzelkomponenten überaus deutlich, aber auch die Summe der Einzeleffekte von

3 µg Tiotropium Bromid und 3 µg Formoterol FU. Sie übertrifft, die Effekte von 10 µg Tiotropium Bromid bzw. 10 µg Formoterol Fumarat (vgl. Abbildung 2).

Tiotropium Bromid alleine hat sowohl mit 3 µg als auch mit 10 µg keinerlei Einfluß auf die Herzfrequenz. Formoterol FU steigert sie hingegen dosisabgestuft und vor allem mit der hohen Dosierung maximal um über 90 %. Auch am Versuchsende werden noch Werte von über 80 % gemessen. Mit den Kombinationen 3 + 3 µg, aber auch 10 + 10 µg Tiotropium Bromid und Formoterol Fumarat sind die Frequenzeffekte deutlich abgeschwächt und liegen unter 30 %.

Beurteilung

Mit der Kombination des Anticholinergikums mit dem β-Mimetikum werden gegenüber den Einzelstoffen völlig überraschende Befunde erhoben:

1. der schnelle Wirkungseintritt
2. die lange Wirkungsdauer

vor allem aber

3. die überadditive bronchospasmolytische Wirkung und
4. die deutlich geringeren Frequenzanstiege, vor allem mit der hohen Formoteroldosis.

Mit dem Kombinationspräparat kann eine deutlich verbesserte therapeutische Wirksamkeit sowohl bei COPD als auch bei Asthma erwartet werden, verbunden mit dem Vorteil der geringeren kardialen Nebenwirkungen.

Tabellen

Tabelle 1: Einfluß von 3 $\mu$g Tiotropium Bromid auf die Herzfrequenz narkotisierter Hunde nach inhalativer Applikation mittels Respimat®, n = 6.

| Herzfrequenz (Schläge/min.) | | | | | | | | | |
|---|---|---|---|---|---|---|---|---|---|
| | **Kontrolle** | **Minuten nach Applikation** | | | | | | | |
| | | **1** | **5** | **10** | **20** | **30** | **60** | **120** | **180** |
| | 66,50 | 63,00 | 67,00 | 64,00 | 61,00 | 63,00 | 67,00 | 63,00 | 66,00 |
| | 87,50 | 87,00 | 84,00 | 82,00 | 87,00 | 81,00 | 89,00 | 87,00 | 87,00 |
| | 86,50 | 84,00 | 84,00 | 89,00 | 89,00 | 89,00 | 84,00 | 77,00 | 86,00 |
| | 109,50 | 115,00 | 115,00 | 116,00 | 120,00 | 121,00 | 104,00 | 105,00 | 105,00 |
| | 110,50 | 119,00 | 119,00 | 118,00 | 110,00 | 110,00 | 111,00 | 110,00 | 100,00 |
| | 85,50 | 85,00 | 87,00 | 90,00 | 93,00 | 97,00 | 97,00 | 92,00 | 96,00 |
| **Mittelwert** | 91,00 | 92,17 | 92,67 | 93,17 | 93,33 | 93,50 | 92,00 | 89,00 | 90,00 |
| **sem** | 6,80 | 8,63 | 8,23 | 8,45 | 8,35 | 8,46 | 6,40 | 7,14 | 5,66 |

| 3 $\mu$g Tiotropium Bromid, % Änderung | | | | | | | | | |
|---|---|---|---|---|---|---|---|---|---|
| | **Kontrolle** | **Minuten nach Applikation** | | | | | | | |
| | | **1** | **5** | **10** | **20** | **30** | **60** | **120** | **180** |
| | 66,50 | -5,26 | 0,75 | -3,76 | -8,27 | -5,26 | 0,75 | -5,26 | -0,75 |
| | 87,50 | -0,57 | -4,00 | -6,29 | -0,57 | -7,43 | 1,71 | -0,57 | -0,57 |
| | 86,50 | -2,89 | -2,89 | 2,89 | 2,89 | 2,89 | -2,89 | -10,98 | -0,58 |
| | 109,50 | 5,02 | 5,02 | 5,94 | 9,59 | 10,50 | -5,02 | -4,11 | -4,11 |
| | 110,50 | 7,69 | 7,69 | 6,79 | -0,45 | -0,45 | 0,45 | -0,45 | -9,50 |
| | 85,50 | -0,58 | 1,75 | 5,26 | 8,77 | 13,45 | 13,45 | 7,60 | 12,28 |
| **Mittelwert** | 91,00 | 0,57 | 1,39 | 1,81 | 1,99 | 2,28 | 1,41 | -2,30 | -0,54 |
| **sem** | 6,80 | 1,99 | 1,83 | 2,25 | 2,72 | 3,42 | 2,62 | 2,53 | 2,93 |

Tabelle 2: Einfluß von 10 $\mu$g Tiotropium Bromid auf die Herzfrequenz narkotisierter Hunde nach inhalativer Applikation mittels Respimat®, n = 6.

| Herzfrequenz (Schläge/min.) | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 66,50 | 79,00 | 75,00 | 75,00 | 77,00 | 79,00 | 74,00 | 75,00 | 70,00 |
| | 87,50 | 96,00 | 91,00 | 88,00 | 89,00 | 90,00 | 85,00 | 83,00 | 83,00 |
| | 86,50 | 85,00 | 80,00 | 79,00 | 77,00 | 76,00 | 75,00 | 76,00 | 87,00 |
| | 109,50 | 104,00 | 102,00 | 101,00 | 101,00 | 101,00 | 103,00 | 103,00 | 105,00 |
| | 110,50 | 102,00 | 102,00 | 102,00 | 101,00 | 96,00 | 101,00 | 102,00 | 101,00 |
| | 85,50 | 76,00 | 75,00 | 76,00 | 77,00 | 74,00 | 73,00 | 74,00 | 74,00 |
| **Mittelwert** | 91,00 | 90,33 | 87,50 | 86,83 | 87,00 | 86,00 | 85,17 | 85,50 | 86,67 |
| **sem** | 6,80 | 4,89 | 5,17 | 5,00 | 4,82 | 4,60 | 5,61 | 5,53 | 5,75 |

| 10 $\mu$g Tiotropium Bromid, % Änderung | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 66,50 | 18,80 | 12,78 | 12,78 | 15,79 | 18,80 | 11,28 | 12,78 | 5,26 |
| | 87,50 | 9,71 | 4,00 | 0,57 | 1,71 | 2,86 | -2,86 | -5,14 | -5,14 |
| | 86,50 | -1,73 | -7,51 | -8,67 | -10,98 | -12,14 | -13,29 | -12,14 | 0,58 |
| | 109,50 | -5,02 | -6,85 | -7,76 | -7,76 | -7,76 | -5,94 | -5,94 | -4,11 |
| | 110,50 | -7,69 | -7,69 | -7,69 | -8,60 | -13,12 | -8,60 | -7,69 | -8,60 |
| | 85,50 | -11,11 | -12,28 | -11,11 | -9,94 | -13,45 | -14,62 | -13,45 | -13,45 |
| **Mittelwert** | 91,00 | 0,49 | -2,93 | -3,65 | -3,30 | -4,14 | -5,67 | -5,26 | -4,24 |
| **sem** | 6,80 | 4,68 | 3,84 | 3,66 | 4,25 | 5,23 | 3,84 | 3,86 | 2,70 |

Tabelle 3: Einfluß von 3 $\mu$g Formoterol Fumarat auf die Herzfrequenz narkotisierter Hunde nach inhalativer Applikation mittels Respimat®, n = 6.

| Herzfrequenz (Schläge/min.) | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 94,50 | 102,00 | 105,00 | 129,00 | 134,00 | 138,00 | 134,00 | 115,00 | 108,00 |
| | 133,00 | 123,00 | 140,00 | 162,00 | 165,00 | 159,00 | 153,00 | 147,00 | 140,00 |
| | 60,00 | 67,00 | 64,00 | 100,00 | 95,00 | 89,00 | 86,00 | 88,00 | 86,00 |
| | 80,50 | 91,00 | 95,00 | 110,00 | 100,00 | 95,00 | 94,00 | 94,00 | 96,00 |
| | 106,50 | 129,00 | 137,00 | 138,00 | 141,00 | 145,00 | 140,00 | 130,00 | 130,00 |
| | 92,50 | 107,00 | 116,00 | 125,00 | 126,00 | 128,00 | 128,00 | 120,00 | 120,00 |
| Mittelwert | 94,50 | 103,17 | 109,50 | 127,33 | 126,83 | 125,67 | 122,50 | 115,67 | 113,33 |
| sem | 10,03 | 9,19 | 11,59 | 8,89 | 10,71 | 11,44 | 10,87 | 9,02 | 8,39 |

| 3 $\mu$g Formoterol Fumarat, % Änderung | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 94,50 | 7,94 | 11,11 | 36,51 | 41,80 | 46,03 | 41,80 | 21,69 | 14,29 |
| | 133,00 | -7,52 | 5,26 | 21,80 | 24,06 | 19,55 | 15,04 | 10,53 | 5,26 |
| | 60,00 | 11,67 | 6,67 | 66,67 | 54,33 | 48,33 | 43,33 | 46,67 | 43,33 |
| | 80,50 | 13,04 | 18,01 | 36,65 | 24,44 | 18,01 | 16,77 | 16,77 | 19,25 |
| | 106,50 | 21,13 | 28,64 | 29,58 | 32,39 | 36,15 | 31,46 | 22,07 | 22,07 |
| | 92,50 | 15,68 | 25,41 | 35,14 | 36,22 | 38,38 | 38,38 | 29,73 | 29,73 |
| Mittelwert | 94,50 | 10,32 | 15,85 | 37,72 | 36,17 | 34,41 | 31,13 | 24,58 | 22,32 |
| sem | 10,03 | 3,99 | 3,99 | 6,24 | 5,25 | 5,28 | 5,10 | 5,12 | 5,36 |

Tabelle 4: Einfluß von 10 $\mu$g Formoterol Fumarat auf die Herzfrequenz narkotisierter Hunde nach inhalativer Applikation mittels Respimat®, n = 6.

| Herzfrequenz (Schläge/min.) | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 94,50 | 116,00 | 153,00 | 155,00 | 157,00 | 159,00 | 163,00 | 176,00 | 152,00 |
| | 133,00 | 145,00 | 136,00 | 191,00 | 204,00 | 207,00 | 210,00 | 209,00 | 205,00 |
| | 60,00 | 109,00 | 146,00 | 152,00 | 153,00 | 150,00 | 149,00 | 146,00 | 141,00 |
| | 80,50 | 96,00 | 120,00 | 144,00 | 156,00 | 156,00 | 140,00 | 140,00 | 130,00 |
| | 106,50 | 105,00 | 120,00 | 160,00 | 158,00 | 150,00 | 150,00 | 145,00 | 145,00 |
| | 92,50 | 122,00 | 122,00 | 130,00 | 135,00 | 140,00 | 140,00 | 135,00 | 135,00 |
| Mittelwert | 94,50 | 115,50 | 132,83 | 155,33 | 160,50 | 160,33 | 158,67 | 158,50 | 151,33 |
| sem | 10,03 | 6,94 | 5,88 | 8,32 | 9,38 | 9,70 | 10,83 | 11,68 | 11,18 |

| 10 $\mu$g Formoterol Fumarat, % Änderung | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 94,50 | 22,75 | 61,90 | 64,02 | 66,14 | 68,25 | 72,49 | 86,24 | 60,85 |
| | 133,00 | 9,02 | 2,26 | 43,61 | 53,38 | 55,64 | 57,89 | 57,14 | 54,14 |
| | 60,00 | 81,67 | 143,33 | 153,33 | 155,00 | 150,00 | 148,33 | 143,33 | 135,00 |
| | 80,50 | 19,25 | 49,07 | 78,88 | 93,79 | 93,79 | 73,91 | 73,91 | 61,49 |
| | 106,50 | -1,41 | 12,68 | 50,23 | 48,36 | 40,85 | 40,85 | 36,15 | 36,15 |
| | 92,50 | 31,89 | 31,89 | 40,54 | 45,95 | 51,35 | 51,35 | 45,95 | 45,95 |
| Mittelwert | 94,50 | 27,20 | 50,19 | 71,77 | 77,10 | 76,65 | 74,14 | 73,79 | 65,59 |
| sem | 10,03 | 11,86 | 20,70 | 17,32 | 17,15 | 16,44 | 15,70 | 15,77 | 14,42 |

Tabelle 5: Einfluß der Kombination von 3 $\mu$g Tiotropium BR + 3 $\mu$g Formoterol FU auf die Herzfrequenz narkotisierter Hunde nach inhalativer Applikation mittels Respimat®, n = 6.

| Herzfrequenz (Schläge/min.) | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 107,50 | 107,00 | 110,00 | 112,00 | 110,00 | 110,00 | 110,00 | 106,00 | 106,00 |
| | 143,00 | 153,00 | 162,00 | 160,00 | 158,00 | 154,00 | 161,00 | 146,00 | 145,00 |
| | 95,00 | 106,00 | 109,00 | 111,00 | 121,00 | 119,00 | 108,00 | 114,00 | 107,00 |
| | 95,50 | 110,00 | 117,00 | 129,00 | 128,00 | 130,00 | 129,00 | 123,00 | 123,00 |
| | 112,00 | 127,00 | 120,00 | 115,00 | 115,00 | 104,00 | 112,00 | 107,00 | 96,00 |
| | 101,50 | 100,00 | 110,00 | 110,00 | 112,00 | 114,00 | 110,00 | 101,00 | 95,00 |
| **Mittelwert** | 109,08 | 117,17 | 121,33 | 122,83 | 124,00 | 121,83 | 121,67 | 116,17 | 112,00 |
| **sem** | 7,31 | 8,07 | 8,33 | 7,69 | 7,31 | 7,37 | 8,47 | 6,73 | 7,78 |

| 3 $\mu$g Tiotropium Bromid + 3 $\mu$g Formoterol Fumarat, % Änderung | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 107,50 | -0,47 | 2,33 | 4,19 | 2,33 | 2,33 | 2,33 | -1,40 | -1,40 |
| | 143,00 | 6,99 | 13,29 | 11,89 | 10,49 | 7,69 | 12,59 | 2,10 | 1,40 |
| | 95,00 | 11,58 | 14,74 | 16,84 | 27,37 | 25,26 | 13,68 | 20,00 | 12,63 |
| | 95,50 | 15,18 | 22,51 | 35,08 | 34,03 | 36,13 | 35,08 | 28,80 | 28,80 |
| | 112,00 | 13,39 | 7,14 | 2,68 | 2,68 | -7,14 | 0,00 | -4,46 | -14,29 |
| | 101,50 | -1,48 | 8,37 | 8,37 | 10,34 | 12,32 | 8,37 | -0,49 | -6,40 |
| **Mittelwert** | 109,08 | 7,53 | 11,40 | 13,17 | 14,54 | 12,76 | 12,01 | 7,42 | 3,46 |
| **sem** | 7,31 | 2,91 | 2,87 | 4,86 | 5,38 | 6,41 | 5,12 | 5,55 | 6,23 |

Tabelle 6: Einfluß der Kombination von 10 $\mu$g Tiotropium Bromid + 10 $\mu$g Formoterol Fumarat auf die Herzfrequenz narkotisierter Hunde nach inhalativer Applikation mittels Respimat®, n = 4.

| Herzfrequenz (Schläge/min.) | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 107,50 | 107,00 | 107,00 | 114,00 | 117,00 | 117,00 | 117,00 | 116,00 | 119,00 |
| | 143,00 | 150,00 | 154,00 | 171,00 | 180,00 | 182,00 | 181,00 | 168,00 | 168,00 |
| | 95,00 | 107,00 | 107,00 | 116,00 | 124,00 | 127,00 | 125,00 | 122,00 | 126,00 |
| | 95,50 | 116,00 | 117,00 | 120,00 | 127,00 | 129,00 | 130,00 | 120,00 | 123,00 |
| **Mittelwert** | 110,25 | 120,00 | 121,25 | 130,25 | 137,00 | 138,75 | 138,25 | 131,50 | 134,00 |
| **sem** | 11,29 | 10,22 | 11,17 | 13,64 | 14,49 | 14,65 | 14,50 | 12,23 | 11,42 |

| 10 $\mu$g Tiotropium Bomid + 10 $\mu$g Formoterol Fumarat, % Änderung | | | | | | | | | |
|---|---|---|---|---|---|---|---|---|---|
| | Kontrolle | Minuten nach Applikation | | | | | | | |
| | | 1 | 5 | 10 | 20 | 30 | 60 | 120 | 180 |
| | 107,50 | -0,47 | -0,47 | 6,05 | 8,84 | 8,84 | 8,84 | 7,91 | 10,70 |
| | 143,00 | 4,90 | 7,69 | 19,58 | 25,87 | 27,27 | 26,57 | 17,48 | 17,48 |
| | 95,00 | 12,36 | 12,36 | 22,11 | 30,53 | 33,68 | 31,58 | 28,42 | 32,63 |
| | 95,50 | 21,47 | 22,51 | 25,65 | 32,98 | 35,08 | 36,13 | 25,65 | 28,80 |
| **Mittelwert** | 110,25 | 9,63 | 10,59 | 18,35 | 24,56 | 26,22 | 25,78 | 19,87 | 22,40 |
| **sem** | 11,29 | 4,77 | 4,80 | 4,29 | 5,44 | 6,04 | 5,97 | 4,61 | 5,06 |

Abbildungen

Fig. 1 zeigt den Einfluß von 3 µg Formoterol Fumarat, 3 µg Tiotropium Bromid sowie der Kombination 3 µg Tiotropium Bromid + 3 µg Formoterol Fumarat auf den Bronchialwiderstand narkotisierter Hunde, n=6.

Fig. 2 zeigt den Einfluß von 10 µg Formoterol Fumarat, 10 µg Tiotropium Bromid sowie der Kombination 3 µg Tiotropium Bromid + 3 µg Formoterol Fumarat auf den Bronchialwiderstand narkotisierter Hunde, n = 6.

**Patentansprüche**

1. Verwendung eines langwirksamen Anticholinergikums zur Herstellung eines ein langwirksames β-Mimetikum enthaltenden Medikaments zur Behandlung von Atemwegserkrankungen unter gleichzeitiger Verringerung der kardialen Nebenwirkungen des β-Mimetikums.

2. Verwendung nach Anspruch 1, wobei es sich bei den Atemwegserkrankungen um Asthma oder COPD handelt.

3. Verwendung nach einem der Ansprüche 1 oder 2, wobei als langwirksames Anticholinergikum Glycopyrroniumbromid oder Verbindungen der Formel (I),

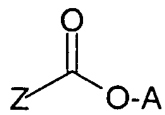
(I)

worin

A für einen Rest der allgemeinen Formel (II)

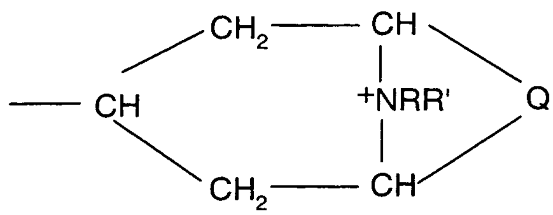
(II)

steht,
in dem

Q eine der zweibindigen Gruppen $-CH_2-CH_2-$, $-CH_2-CH_2-CH_2-$, $-CH=CH-$, oder

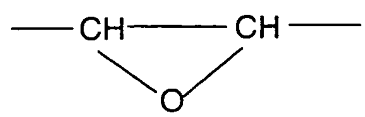
;

R ein gegebenenfalls halogen- oder hydroxysustituierter $C_1$-$C_4$-Alkylrest,
R' ein $C_1$-$C_4$-Alkylrest bedeutet und R und R' gemeinsam auch einen $C_4$-$C_6$-Alkylenrest bilden können, und
der positiven Ladung des N-Atoms ein Äquivalent eines Anions X gegenübersteht,

Z für eine der Gruppen

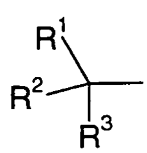
(III) oder

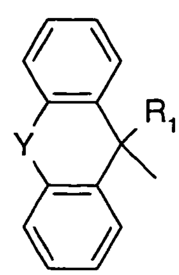
(IV)

steht,
worin

Y eine Einfachbindung, ein O- oder S-Atom oder eine der Gruppen $-CH_2-$, $-CH_2-CH_2-$, -CH=CH-, $-OCH_2-$ oder $-SCH_2-$ repräsentiert;

$R^1$ Wasserstoff, OH, $C_1$-$C_4$-Alkoxy oder $C_1$-$C_4$-Alkyl, welches gegebenenfalls durch Hydroxy substituiert sein kann;

$R^2$ ein Thienyl-, Phenyl-, Furyl-, Cyclopentyl- oder Cyclohexylrest, wobei diese Reste auch methylsubstituiert, Thienyl und Phenyl auch fluor- oder chlorsubstitueniert sein können,

$R^3$ Wasserstoff oder ein Thienyl- oder Phenylrest, der gegebenenfalls durch Halogen oder $C_1$-$C_4$-Alkyl substituiert sein kann, bedeuten,

gegebenenfalls in Form ihrer Racemate, ihrer Enantiomere, ihrer Diastereomere und ihrer Gemische, in Betracht kommen.

**4.** Verwendung nach einem der Ansprüche 1 bis 3, **dadurch gekennzeichnet, daß** das langwirksame Anticholinergikum ausgewählt ist Glycopyrroniumbromid oder Verbindungen der Formel (I), worin

A für einen Rest der allgemeinen Formel (II)

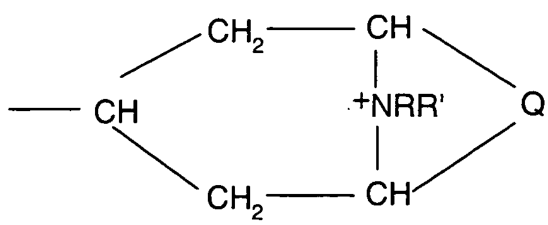

(II)

steht,
in dem

Q eine der zweibindigen Gruppen -CH = CH-, $-CH_2-CH_2-$ oder

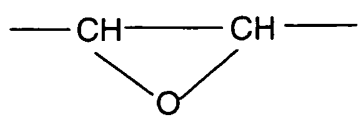

;

R Methyl oder Ethyl;
R' Methyl,
und
der positiven Ladung des N-Atoms ein Äquivalent des Anions X = Bromid gegenübersteht,

Z für eine der Gruppen

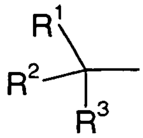 (III) oder 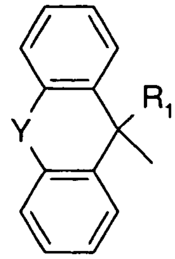 (IV)

steht,

worin

Y ein O-Atom;
$R^1$ Wasserstoff, OH oder Hydroxymethyl;
$R^2$ ein Thienyl-, Phenyl-, oder Cyclohexylrest;
$R^3$ Wasserstoff, Thienyl oder Phenylrest,

gegebenenfalls in Form ihrer Racemate, ihrer Enantiomere, ihrer Diastereomere und ihrer Gemische.

**5.** Verwendung nach einem der Ansprüche 1 bis 4, **dadurch gekennzeichnet, daß** das langwirksame Anticholinergikum ausgewählt ist aus den Salzen des Tiotropiums.

**6.** Verwendung nach einem der Ansprüche 1 bis 4, **dadurch gekennzeichnet, daß** das langwirksame Anticholinergikum Glycopyrroniumbromid ist, gegebenenfalls in Form seiner Racemate, Enantiomere, Diastereomere oder deren Gemische.

**7.** Verwendung nach einem der Ansprüche 1 bis 6, wobei das langwirksame β-Mimetikum ausgewählt ist aus der Gruppe Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol,
4-Hydroxy-7-[2-{[2-{[3-(2-phenylethoxy)propyl]sulfonyl}ethyl]-amino}ethyl]-2(3H)-benzothiazolon,
1-(2-Fluoro-4-hydroxyphenyl)-2-[4-(1-benzimidazolyl)-2-methyl-2-butylamino]ethanol, 1-[3-(4-Methoxybenzylamino)-4-hydroxyphenyl]-2-[4-(1-benzimidazolyl)-2-methyl-2-butylamino]ethanol,
1-[2H-5-Hydroxy-3-oxo-4H-1,4-benzoxazin-8-yl]-2-[3-(4-N,N-dimethylaminophenyl)-2-methyl-2-propylamino] ethanol,
1-[2H-5-Hydroxy-3-oxo-4H-1,4-benzoxazin-8-yl]-2-[3-(4-methoxyphenyl)-2-methyl-2-propylamino]ethanol,
1-[2H-5-Hydroxy-3-oxo-4H-1,4-benzoxazin-8-yl]-2-[3-(4-n-butyloxyphenyl)-2-methyl-2-propylamino]ethanol,
1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol,
5-Hydroxy-8-(1-hydroxy-2-isopropylaminobutyl)-2H-1,4-benzoxazin-3-(4H)-on,
1-(4-Amino-3-chloro-5-trifluormethylphenyl)-2-*tert*.-butylamino)ethanol und
1-(4-Ethoxycarbonylamino-3-cyano-5-fluorophenyl)-2-(*tert*.-butylamino)ethanol,
gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**8.** Verwendung nach einem der Ansprüche 1 bis 7, wobei das langwirksame β-Mimetikum ausgewählt ist aus Formoterol und Salmeterol, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**9.** Verwendung nach einem der Ansprüche 1 bis 8, **dadurch gekennzeichnet, daß** das langwirksame Anticholinergikum ausgewählt ist aus den Salzen des Tiotropiums und dass das langwirksame β-Mimetikum ausgewählt ist aus Formoterol und Salmeterol, gegebenenfalls in Form ihrer Racemate, ihrer Enantiomere, ihrer Diastereomere und ihrer Gemische, sowie gegebenenfalls ihrer pharmakologisch unbedenklichen Säureadditionssalze.

**10.** Verwendung nach einem der Ansprüche 1 bis 9, **dadurch gekennzeichnet, daß** das langwirksame Anticholinergikum ausgewählt ist aus den Salzen des Tiotropiums und dass das langwirksame β-Mimetikum Salmeterol ist, gegebenenfalls in Form seiner Racemate, seiner Enantiomere und Gemische, sowie gegebenenfalls seiner pharmakologisch unbedenklichen Säureadditionssalze.

**11.** Verwendung nach einem der Ansprüche 1 bis 10, **dadurch gekennzeichnet, dass** es sich bei dem Medikament um ein Pulver für die Inhalation handelt.

**12.** Medikament, enthaltend als langwirksames Anticholinergikum ein Salz des Tiotropiums, als langwirksames β-Mimetikum Salmeterol, gegebenenfalls in Form seiner Racemate, seiner Enantiomere, und Gemische davon, sowie gegebenenfalls in Form seiner pharmakologisch unbedenklichen Säureadditionssalze sowie pharmakologisch unbedenkliche Träger- oder Hilfsstoffe.

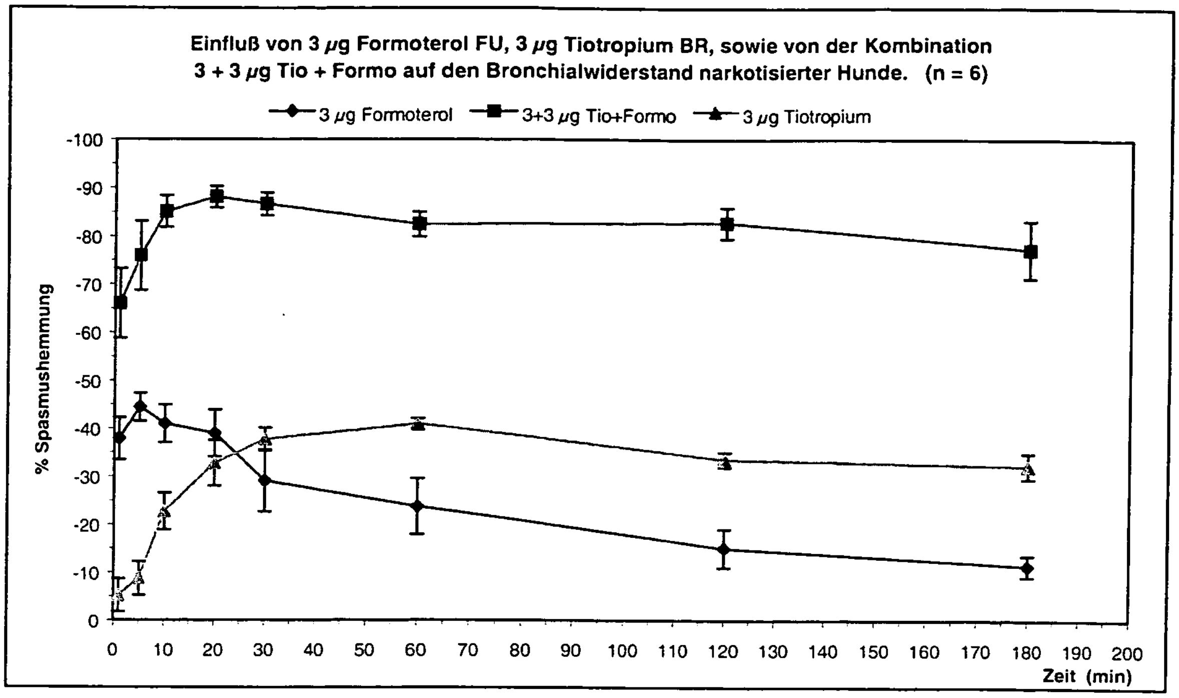
Einfluß von 3 µg Formoterol FU, 3 µg Tiotropium BR, sowie von der Kombination
3 + 3 µg Tio + Formo auf den Bronchialwiderstand narkotisierter Hunde. (n = 6)
3 µg Formoterol
3+3 µg Tio+Formo
3 µg Tiotropium
% Spasmushemmung
-100
-90
-80
-70
-60
-50
-40
-30
-20
-10
0
0 10 20 30 40 50 60 70 80 90 100 110 120 130 140 150 160 170 180 190 200
Zeit (min)

Fig. 1

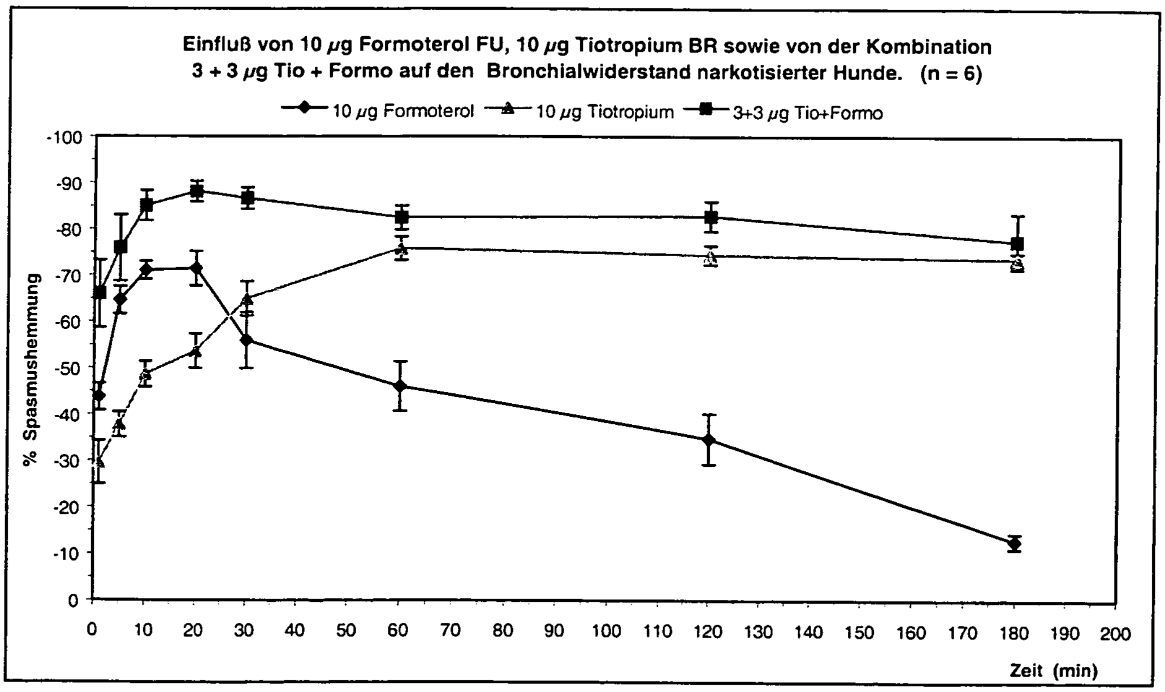
Einfluß von 10 µg Formoterol FU, 10 µg Tiotropium BR sowie von der Kombination
3 + 3 µg Tio + Formo auf den Bronchialwiderstand narkotisierter Hunde. (n = 6)
10 µg Formoterol
10 µg Tiotropium
3+3 µg Tio+Formo
% Spasmushemmung
-100
-90
-80
-70
-60
-50
-40
-30
-20
-10
0
0 10 20 30 40 50 60 70 80 90 100 110 120 130 140 150 160 170 180 190 200
Zeit (min)

Fig. 2